# EUROPÄISCHE PATENTSCHRIFT

(11) **EP 3 219 747 B1**
(45) Veröffentlichungstag und Bekanntmachung des Hinweises auf die Patenterteilung: **17.10.2018**
(21) Anmeldenummer: 16160521.7
(22) Anmeldetag: 15.03.2016
(51) Int. Cl.: C08H 7/00, C12P 7/22, C04B 24/18

(54) **ENZYMATISCH MODIFIZIERTE LIGNINE**
ENZYMATICALLY MODIFIED LIGNINS
LIGNINE MODIFIEE PAR ENZYME

(43) Veröffentlichungstag der Anmeldung: 20.09.2017
(73) Patentinhaber: Sika Technology AG, 6340 Baar (CH); EMPA Eidgenössische Materialprüfungs- und Forschungsanstalt, 8600 Dübendorf (CH)
(72) Erfinder: Schober, Irene, 8008 Zürich (CH); Richter, Michael, 94315 Straubing (DE); Heck, Tobias, 3006 Bern (CH); Jankowska, Dagmara, 9000 St. Gallen (CH)
(74) Vertreter: Sika Patent Attorneys

(56) Entgegenhaltungen:
- EP-A1- 0 367 389
- EP-A1- 1 040 145
- US-A- 4 703 801

## Beschreibung

### Technisches Gebiet

Die Erfindung betrifft enzymatisch modifizierte Lignine, ein Verfahren zu deren Herstellung und deren Verwendung als Dispergiermittel für mineralische Bindemittel.

### Stand der Technik

Lignin ist nach Zellulose das am weitesten verbreitete natürliche Polymer weltweit. Es fällt als Nebenprodukt bei der Zellstoffproduktion an und wird zum Grossteil zur Energie- und Rohstoffrückgewinnung verbrannt. Nur ca. 5% der Zellstofffabriken arbeiten mit dem Sulfitprozess, bei dem das wasserlösliche und als Dispergiermittel verwendbare Ligninsulfonat, auch Lignosulfonat genannt, anfällt. Da ca. 95% der Zellstoff-Fabriken nach dem Kraft-Prozess arbeiten fällt mehrheitlich das wasserunlösliche Kraft-Lignin an. Auch in Bioraffinerien, in denen Holz und Lignocellulose zu Bioethanol umgewandelt werden, entsteht als Nebenprodukt Lignin. In den meisten Fällen wird Lignin allerdings zur Energiegewinnung verbrannt. Es ist aber eine höherwertige Verwertung dieses nachwachsenden Rohstoffes wünschenswert. Um Lignin als Dispergiermittel in wässrigen Medien nutzen zu können, muss es wasserlöslich sein oder durch Modifikation wasserlöslich gemacht werden.

EP 0 367 389 beschreibt die Herstellung eines Dispergiermittels durch Umsetzung von Lignin mit Formaldehyd und anschliessender Sulfonierung. Dabei werden toxische Chemikalien wie Formaldehyd und Schwefeldioxid und hohe Reaktionstemperaturen eingesetzt.

EP 0 669 953 beansprucht ein Verfahren zur Herstellung von Polymeren, die Lignin und organische Verbindungen enthalten. Es gibt dabei keine Hinweise, dass die Reaktionsprodukte bei pH 7 oder darunter wasserlöslich sind oder als Dispergiermittel geeignet sind.

EP 1 040 145 beschreibt die enzymatische Pfropfung von ethylenisch ungesättigten Monomeren auf Lignin mit radikalisch oxidierenden Enzymen in Gegenwart von organischen Peroxiden oder Hydroperoxiden. Es gibt dabei keine Hinweise, dass die erhaltenen Produkte in Wasser bei pH 7 oder darunter löslich sind.

### Darstellung der Erfindung

Aufgabe der vorliegenden Erfindung ist es, wasserlösliche modifizierte Lignine zur Verfügung zu stellen, welche in einem einfachen Verfahren bei milden Bedingungen aus wasserunlöslichen Ligninen durch Umsetzung mit organischen Verbindungen herstellbar sind und als Dispergiermittel für mineralische Bindemittelzusammensetzungen eingesetzt werden können.

Überraschenderweise wird diese Aufgabe durch ein wasserlösliches modifiziertes Lignin wie in Anspruch 1 beschrieben gelöst. Es ist auf einfache Weise bei milden Bedingungen und ohne toxische Chemikalien, wie beispielsweise Formaldehyd, durch enzymatische Umsetzung aus wasserunlöslichem Lignin und mindestens einer organischen Verbindung herstellbar. Dabei beruht das wasserlösliche modifizierte Lignin weitgehend auf nachwachsenden Rohstoffen, was bei vielen Anwendungen erwünscht ist, und ist einfach handhabbar.

Die Umsetzung wird durch Enzyme katalysiert. Sie kann somit unter milden Bedingungen erfolgen, wobei keine Aldehyde, Ketone oder ähnliche Verbindungen zur Verknüpfung der organischen Verbindung mit dem Lignin benötigt werden, wie im Stand der Technik bei der chemischen Ligninmodifikation sonst üblich. Dies ist vorteilhaft, da die meisten Aldehyde, speziell Formaldehyd, oder Ketone, die hierfür verwendet werden, gesundheitlich bedenkliche Stoffe sind.

Das erfindungsgemässe wasserlösliche modifizierte Lignin ist hervorragend geeignet als Dispergiermittel für mineralische Bindemittel. Dabei übt es eine deutliche Wirkung als Verflüssiger aus, wodurch der Wassergehalt einer mineralischen Bindemittel-Zusammensetzung bei gleicher Verarbeitbarkeit gesenkt werden kann, was sehr erwünscht ist. Überraschenderweise bewirkt es zusätzlich zur Wirkung als Verflüssiger eine deutlich geringere Verzögerung des Abbindens der mineralischen Bindemittel-Zusammensetzung verglichen mit dem für die Herstellung eingesetzten wasserunlöslichen Lignin, was ebenfalls sehr erwünscht ist. Die damit hergestellten ausgehärteten mineralischen Zusammensetzungen weisen unverändert gute mechanische Eigenschaften auf.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein wasserlösliches modifiziertes Lignin **ML,** herstellbar durch enzymatische Umsetzung von mindestens einem wasserunlöslichen Lignin **L** mit mindestens einer organischen Verbindung **V,** welche mindestens eine Gruppe ausgewählt aus primärer oder sekundärer Aminogruppe, Hydroxylgruppe und Phenylgruppe besitzt und ein mittleres Molekulargewicht Mₙ im Bereich von 75 bis 2'500 g/mol aufweist, wobei bei der enzymatischen Umsetzung mindestens ein Enzym, ausgewählt aus Peroxidasen oder Phenoloxidasen, insbesondere Laccasen oder Tyrosinasen, vorhanden ist.

Als "wasserlöslich" wird ein Lignin bezeichnet, bei welchem von 1.0 g des getrockneten Lignins in 100 g deionisiertem Wasser bei 20°C nach 30 Minuten unter Rühren mindestens 95 Gewichts-% gelöst sind.
Als "wasserunlöslich" wird ein Lignin bezeichnet, bei welchem von 1.0 g des getrockneten Lignins in 100 g deionisiertem Wasser bei 20°C nach 30 Minuten unter Rühren weniger als 100 mg (10 Gewichts-%) gelöst sind.
Als "teilweise wasserlöslich" gelten Lignine, die keine dieser Bedingungen erfüllen.
Unter "Molekulargewicht" versteht man im vorliegenden Dokument die molare Masse (in Gramm pro Mol) eines Moleküls. Als "mittleres Molekulargewicht" wird das Gewichtsmittel M_{w} oder das Zahlenmittel Mₙ einer oligomeren oder polymeren Mischung von Molekülen bezeichnet, welches wie in den Beispielen beschrieben bestimmt wird.

Das wasserlösliche modifizierte Lignin **ML** besteht aus einem Anteil, der vom wasserunlöslichen Lignin **L** herrührt und einem Anteil, der von der organischen Verbindung **V** herrührt. Der Anteil, der vom wasserunlöslichen Lignin **L** herrührt, beträgt bevorzugt mindestens 20 Gewichts-%, mehr bevorzugt mindestens 40 Gewichts-%, besonders bevorzugt mindestens 50 Gewichts-%.

Das wasserlösliche modifizierte Lignin **ML** hat bevorzugt ein mittleres Molekulargewicht M_{w} im Bereich von 1'000 bis 150'000 g/mol, bevorzugt 1'000 bis 40'000 g/mol, insbesondere 1'000 bis 20'000 g/mol.

Als wasserunlösliches Lignin **L** für die Herstellung des wasserlöslichen Lignins **ML** geeignet sind alle Lignine, unabhängig von Herkunft und Vorbehandlung. Geeignet sind insbesondere sogenannte Kraftlignine, Organosolvlignine, Sodalignine, Säurelignine oder Lignine aus dem enzymatischen Celluloseabbau von ligninhaltigen Stoffen.
Kraftlignine fallen typischerweise bei der Zellstoffproduktion aus Holz nach dem Kraft-Prozess, auch Sulfat-Prozess genannt, an. Die Isolierung des Kraftlignins aus der alkalischen Schwarzlauge kann durch verschiedene Methoden erfolgen. Grosstechnisch eingesetzte Methoden zur Isolierung von Lignin aus der Schwarzlauge sind zum Beispiel der LignoBoost-Prozess oder der LignoForce™-Prozess.
Organosolvlignine fallen typischerweise beim Organosolv-Prozess, der bei der Zellstoffproduktion oder auch in Bioraffinerien eingesetzt werden kann, an. Hier wird das Lignin bei hohen Temperaturen durch ein Wasser-Lösemittel-Gemisch aus Holz oder anderen ligninhaltigen Materialien herausgelöst. Das so isolierte Lignin ist besonders rein. Ein Beispiel für ein solches Lignin ist Lignol HP™von Lignol, Canada.
Sodalignine fallen typischerweise bei der Zellstoffgewinnung mit dem Sodaprozess aus Einjahrespflanzen wie beispielsweise Gräsern oder Stroh an. Ein Beispiel dafür ist das von ALM in Indien hergestellte Biosurfact 8000. Säurelignine entstehen beim Behandeln von ligininhaltigen Stoffen, beispielsweise Holz, Grünschnitt, Algen oder Gräsern, mit starken Säuren. Hierbei werden die durch die Säuren abgebauten oder in Säuren löslichen Stoffe gelöst und das säureunlösliche Lignin kann abgetrennt werden.
Weitere Beispiele zur Gewinnung von geeigneten Ligninen sind die Dampfbehandlung von ligninhaltigen Materialien und anschliessender enzymatischer Abbau der Cellulose.
Es ist dem Fachmann bekannt, dass durch unterschiedliche Gewinnungsverfahren und aus unterschiedlichen ligninhaltigen Rohstoffen die Struktur des Lignins unterschiedlich sein kann, speziell das Molekulargewicht und die Anzahl an funktionellen Gruppen. Zudem können im Lignin auch Verunreinigungen wie beispielsweise Abbauprodukte von Cellulose, Hemicellulosen oder Proteine oder Salze enthalten sein.
Ein für die Erfindung geeignetes wasserunlösliches Lignin **L** stammt insbesondere aus einem ligninhaltigen Material, wie es in der Natur vorkommt, insbesondere Nadelholz, Laubholz oder Stroh. Bevorzugt ist ein Lignin aus Holz oder Stroh. Besonders bevorzugt ist ein Lignin aus Nadelholz oder ein Organosolvlignin aus Laubholz, insbesondere ein Lignin aus Nadelholz.
Es können auch Mischungen unterschiedlicher Lignine eingesetzt werden.

Das wasserunlösliche Lignin **L** kann bei der enzymatischen Umsetzung mit der organischen Verbindung **V** als Pulver oder gelöst, beispielsweise als Na-Salz in alkalischer wässriger Lösung oder gelöst in einer Mischung aus Wasser und Aceton oder Wasser und Dioxan in einem Volumenverhältnis im Bereich von 50:50 bis 80:20, eingesetzt werden. Bevorzugt ist der Einsatz als Pulver oder als Na-Salz in alkalischer wässriger Lösung. Auf diese Weise kann ohne organische Lösemittel gearbeitet werden. Die Zugabe als Pulver weist den Vorteil auf, dass der Schritt des Auflösens des Lignins entfällt. Die Zugabe als Na-Salz weist den Vorteil auf, dass die Reaktionsmischung besonders homogen ist.

Für die Herstellung des wasserlöslichen Lignins **ML** wird eine organische Verbindung **V,** welche mindestens eine Gruppe ausgewählt aus primärer oder sekundärer Aminogruppe, Hydroxylgruppe und Phenylgruppe besitzt und ein mittleres Molekulargewicht Mₙ im Bereich von 75 bis 2'500 g/mol aufweist, eingesetzt.

Bevorzugt ist die organische Verbindung **V** ausgewählt aus der Gruppe bestehend aus
A) Verbindungen der Formel (I),

   Zₓ-R-G-R-W_{y} (I)

   wobei
   Z unabhängig voneinander für -OH, -NH₂, O-R¹ oder R¹ steht,
   G für einen Phenylen- oder Naphthylenrest steht,
   R unabhängig voneinander für eine kovalente Bindung oder einen linearen oder verzweigten, gegebenenfalls ungesättigte Anteile enthaltenden aliphatischen Kohlenwasserstoffrest mit 1 bis 8 C-Atomen, insbesondere für eine kovalente Bindung, steht,
   R¹ unabhängig voneinander für eine Alkylgruppe mit 1 bis 4 C-Atomen steht,
   x für 1 oder 2 oder 3 steht,
   y für 1 oder 2 oder 3 steht, und
   W für -COOM, -SO₃M, -OSO₃M, -PO₃M₂ oder -OPO₃M₂ steht, wobei M für H, ein Alkalimetall, ein Erdalkalimetall, ein zwei- oder dreiwertiges Metall oder ein organisches oder anorganisches Ammonium steht;
B) Verbindungen der Formel (II) 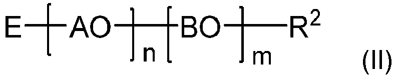 wobei
   E für eine Phenoxy- oder eine primäre Aminogruppe steht,
   A und B unabhängig voneinander für einen Alkylenrest mit 2 bis 4 C-Atomen stehen,
   (n+m) für 1 bis 50 steht, und
   R² für H, eine Alkylgruppe mit 1 bis 8 C-Atomen, -COOM, -SO₃M, -OSO₃M, -PO₃M₂ oder -OPO₃M₂ steht;
C) Aminosäuren, insbesondere eine der 21 natürlichen alpha-Aminocarbonsäuren;
   und
D) aliphatischen, linearen oder verzweigten Aminosulfonsäuren mit 2 bis 10 C-Atomen.

Bevorzugt steht n für 0 bis 50. Bevorzugt steht m für 0 bis 50.

Besitzt die organische Verbindung **V** eine Säuregruppe, so kann diese als freie Säure oder als Salz vorliegen. Im Falle eines Salzes, kann das Kation das Ion eines Alkalimetalls, eines Erdalkalimetalls, eines ein-, zwei- oder dreiwertigen Metalls oder ein organisches oder anorganisches Ammoniums sein. Davon bevorzugt ist Natrium, Kalium oder Kalzium.

Bevorzugte Verbindungen der Formel (I) sind aromatische Verbindungen mit mindestens einer Sulfonsäuregruppe oder Carbonsäuregruppe und mindestens einer an den Phenylen- oder Naphthylen-Rest gebundenen Aminogruppe, insbesondere 4-Amino-3-hydroxybenzoesäure, 3-Amino-4-hydroxybenzoesäure, Anthranilsäure, 4-Aminosalicylsäure, 4-Aminobenzoesäure, Sulfanilsäure, 4-Aminobenzol-1,3-disulfonsäure, 4-Amino-5-hydroxy-2,7-naphthalene-disulfonsäure, 5-Amino-2-methylbenzolsulfonsäure, 4-Amino-3-methylbenzolsulfonsäure oder 6-Amino-3-methylbenzolsulfonsäure.
Davon bevorzugt sind Verbindungen mit einer Sulfonsäuregruppe.
Davon weiterhin bevorzugt ist Sulfanilsäure, 4-Aminobenzoesäure oder 5-Amino-2-methylbenzolsulfonsäure.
Besonders bevorzugt ist Sulfanilsäure oder 4-Aminobenzoesäure.

Bevorzugte Verbindungen der Formel (II) sind Verbindungen der Formel (II), bei welchen E für eine primäre Aminogruppe, A für einen Ethylenrest, B für einen 1,2-Propylenrest, R² für eine Methylgruppe und entweder n für 2 bis 50 und m für 0 oder n für 10 bis 50 und m für 0 bis 10 stehen. Insbesondere geeignet sind Jeffamine® der Firma Huntsman, insbesondere Jeffamine® M-2070. Weiterhin bevorzugte Verbindungen der Formel (II) sind Verbindungen der Formel (II), bei welchen R² für -OPO₃M₂ steht. Dabei können diese Verbindungen neben dem Monoester auch Anteile von Di- und/oder Triester enthalten, wie sie bei der Herstellung von Phosphatestern durch Veresterung eines Alkohols mit Phosphorsäure, Polyphosphorsäure oder Phosphorsäurepentoxid neben dem Monoester ebenfalls entstehen können.

Bevorzugte Aminosäuren sind alpha-Aminocarbonsäuren, insbesondere ausgewählt aus der Gruppe bestehend aus Glycin, Alanin, Glutaminsäure, Asparaginsäure, Tyrosin, Serin, Histidin und Arginin.

Bevorzugte Aminosulfonsäuren sind lineare Aminosulfonsäuren mit 2 bis 4 C-Atomen, insbesondere Taurin.

Für die Herstellung des wasserlöslichen modifizierten Lignins **ML** kann das wasserunlösliche Lignin **L** auch mit einer Mischung aus mehreren organischen Verbindungen **V** enzymatisch umgesetzt werden. Eine solche Mischung kann beispielsweise eine Verbindung der Formel (I) und eine Verbindung der Formel (II) oder eine Aminosäure oder eine Aminosulfonsäure enthalten, oder sie kann mehr als eine Verbindung der Formel (I) oder der Formel (II) oder mehrere Aminosäuren oder Aminosulfonsäuren enthalten.

Die Herstellung des wasserlöslichen Lignins **ML** erfolgt durch enzymatische Umsetzung. Enzyme sind Biokatalysatoren, die auf spezifische Reaktionen spezialisiert sind. Daher sind nicht alle Enzyme geeignet für die beschriebene Umsetzung.
Geeignet sind beispielsweise Peroxidasen oder insbesondere Phenoloxidasen, wie insbesondere Laccasen oder Tyrosinasen.

Bevorzugt ist bei der enzymatischen Umsetzung mindestens ein Enzym ausgewählt aus Laccasen oder Tyrosinasen vorhanden.
Das verwendete Enzym kann bakteriellen, pflanzlichen oder auch pilzlichen Ursprungs sein. Bevorzugt sind Laccasen pilzlichen Ursprungs, insbesondere die Laccase aus Trametes versicolor (erhältlich bei Sigma Aldrich). Weiter bevorzugt sind Laccasen, die auch unter alkalischen Bedingungen ihre Aktivität beibehalten.
Als Tyrosinasen sind insbesondere pilzliche Tyrosinasen (erhältlich bei Sigma Aldrich) geeignet.
Als Peroxidasen sind insbesondere Rettich-Peroxidasen (erhältlich bei Sigma Aldrich) geeignet.

Für die Umsetzung benötigen diese Enzyme Sauerstoff. Bei den Laccasen und Tyrosinasen reicht der vorhandene Luftsauerstoff typischerweise aus. Bei der Verwendung von Peroxidasen ist es vorteilhaft, wenn für die Umsetzung zusätzlich eine Sauerstoffquelle, beispielsweise eine geringe Menge Wasserstoffperoxid, vorhanden ist.
Das Enzym kann in Lösung oder als Pulver, gereinigt oder als Rohextrakt, und/oder immobilisiert auf einem Träger eingesetzt werden.
Das Enzym kann nach Erreichen des gewünschten Reaktionsumsatzes deaktiviert werden, insbesondere durch Erhitzen des Reaktionsgemisches auf mindestens 80°C, bevorzugt mindestens 90°C.

Die Aktivität eines Enzyms wird üblicherweise in U/mg (Units pro Milligramm) oder U/ml (Units pro Milliliter) angegeben. Die Units geben an, wie viele Mol einer bestimmten Testsubstanz pro Minute unter Standardbedingungen umgesetzt werden. Da die Aktivität eines Enzyms von den Testbedingungen wie pH, Testsubstanz und Temperatur abhängt, kann diese stark variieren. Wenn in dieser Anmeldung von U/mg oder U/ml die Rede ist, so wurden die Angaben des Enzymherstellers verwendet; es handelt sich dabei also nicht um die effektiven Aktivitäten unter den Reaktionsbedingungen.

Bei der enzymatischen Umsetzung kann bevorzugt ein Mediator eingesetzt werden. Ein Mediator für enzymatische Reaktionen ist eine Substanz, die durch das Enzym aktiviert wird und die Aktivierung zum Substrat, in diesem Falle zum wasserunlöslichen Lignin **L** und/oder zur organischen Verbindung **V,** transferiert. Dadurch kann die Umsetzung beschleunigt werden. Mediatoren für Laccasen gibt es viele und sie sind dem Fachmann bekannt. Ein geeigneter Mediator für Laccasen ist insbesondere 2,2'-Azino-bis(3-ethylbenzthiazolin-6-sulfonsäure) (ABTS) oder 2,6-Dimethoxyphenol (DMP) oder ein Extrakt von Naturstoffen, insbesondere ein Tee- oder ein Holz-Extrakt, insbesondere in Form eines Heisswasser-Extrakts von Tee bzw. von Holzspänen oder Sägemehl. Bevorzugte Mediatoren für die Umsetzungen mit Laccase sind Extrakte von Schwarztee, Grüntee oder Holz, da diese kostengünstig und ungiftig sind.

Bevorzugt als Reaktionsmedium für die enzymatische Umsetzung zur Herstellung des wasserlöslichen modifizierten Lignins **ML** ist Wasser oder ein Gemisch aus Wasser und einem organischen Lösemittel, insbesondere Aceton oder Dioxan. Der Anteil des organischen Lösemittels in der Reaktionsmischung ist bevorzugt kleiner als 10 Gewichts-%, insbesondere kleiner als 5 Gewichts-%, am meisten bevorzugt wird ganz ohne organische Lösemittel gearbeitet. Für den Fall, dass das wasserunlösliche Lignin **L** gelöst in Wasser/Aceton oder Wasser/Dioxan eingesetzt wird, enthält das Reaktionsmedium geringe Anteile an Aceton bzw. Dioxan.

Bevorzugt wird die enzymatische Umsetzung also in einem Reaktionsmedium enthaltend Wasser und gegebenenfalls mindestens ein organisches Lösemittel durchgeführt, wobei der Lösemittelanteil bevorzugt weniger als 10 Gewichts-%, insbesondere weniger als 5 Gewichts-%, bezogen auf die gesamte Reaktionsmischung beträgt und die Umsetzung am meisten bevorzugt ganz ohne organische Lösemittel erfolgt.

Der pH der Reaktionsmischung bei der enzymatischen Umsetzung kann durch Zugabe von Basen oder Säuren eingestellt werden. Bevorzugte Basen sind Ammoniak, NaOH oder KOH. Bevorzugte Säuren sind anorganische Säuren, wie insbesondere Salzsäure, Schwefelsäure, schwefelige Säure, Phosphorsäure oder phosphorige Säure, oder organische Säuren, wie insbesondere Ameisensäure oder Essigsäure oder Alkylsulfonsäuren. Es können auch Pufferlösungen wie insbesondere Tris-HCI Puffer oder universelle Puffer wie insbesondere Mcllvaine oder Britton Robinson Puffer eingesetzt werden. Für den Fall, dass die organische Verbindung **V** eine Säure- und/oder eine Aminogruppe aufweist, kann diese ganz oder teilweise für die Einstellung des pH-Wertes bei der enzymatischen Umsetzung genutzt werden.
Die enzymatische Umsetzung wird bevorzugt bei milden Bedingungen durchgeführt. Insbesondere wird sie bei einem pH-Wert im Bereich von 2 bis 11, bevorzugt 5 bis 10, insbesondere 6 bis 10, durchgeführt. Da viele Laccasen ab pH 8 nicht mehr aktiv sind, ist für solche Laccasen eine Umsetzung bei einem pH-Wert im Bereich von 6 bis 8 bevorzugt. Für alkalistabile Laccasen ist eine Umsetzung bei einem pH-Wert im Bereich von 8 bis 10 bevorzugt. Für Peroxidasen und Tyrosinasen liegt der bevorzugte pH-Wert im Bereich von 6 bis 9.

Bei der enzymatischen Umsetzung für die Herstellung des wasserlöslichen modifizierten Lignins **ML** werden pro Gramm wasserunlösliches Lignin **L** bevorzugt 0.4 bis 15 mmol, besonders bevorzugt 0.8 bis 10 mmol, insbesondere 1 bis 5 mmol, am meisten bevorzugt 1 bis 3 mmol, der organischen Verbindung **V** eingesetzt.

Die geeignete Menge Enzym für die enzymatische Umsetzung variiert je nach Enzymtyp, Reaktionsbedingungen, pH-Wert und Typ und Menge an organischer Verbindung **V.** Das Enzym wird bevorzugt in einer solchen Menge eingesetzt, dass das wasserunlösliche Lignin **L** in weniger als 3 Tagen, bevorzugt in weniger als 2 Tagen, insbesondere in weniger als 24 Stunden, zum wasserlöslichen modifizierten Lignin **ML** umgesetzt ist.

Die geeignete Menge wasserunlösliches Lignin **L** in der Reaktionsmischung hängt von den Reaktionsbedingungen und der jeweiligen organischen Verbindung **V** ab. Sie ist nach oben dadurch begrenzt, dass die Reaktionsmischung noch rührbar sein soll.

Werden Laccasen oder Tyrosinasen als Enzyme eingesetzt, wird die enzymatische Umsetzung entweder so stark gerührt, dass Luftsauerstoff für die Reaktion zur Verfügung steht, oder es kann zeitweise oder kontinuierlich Luft durch die Lösung geführt werden, beispielsweise in Form von Pressluft. Werden Peroxidasen als Enzyme eingesetzt, so wird der Lösung bevorzugt Wasserstoffperoxid in einer solchen Menge zugegeben, dass die Konzentration von H₂O₂ in der Lösung unter 1 mM, bevorzugt unter 0.1 mM, bleibt.

Ein weiterer Gegenstand der Erfindung ist ein Verfahren zur Herstellung des wasserlöslichen modifizierten Lignins **ML,** wie vorgängig beschrieben, indem ein wasserunlösliches Lignin **L** mit mindestens einer organischen Verbindung **V** enzymatisch umgesetzt wird, wobei die organische Verbindung **V** mindestens eine Gruppe ausgewählt aus primärer oder sekundärer Aminogruppe, Hydroxylgruppe und Phenylgruppe besitzt und ein mittleres Molekulargewicht Mₙ im Bereich von 75 bis 2'500 g/mol aufweist, wobei bei der enzymatischen Umsetzung mindestens ein Enzym, ausgewählt aus Peroxidasen oder Phenoloxidasen, insbesondere Laccasen oder Tyrosinasen, vorhanden ist Dieses Verfahren wird bevorzugt in der bereits beschriebenen Weise durchgeführt.

Aus dem beschriebenen Verfahren wird das wasserlösliche modifizierte Lignin **ML** als Bestandteil eines Reaktionsprodukts erhalten, wobei als "Reaktionsprodukt" das Reaktionsgemisch am Ende der enzymatischen Umsetzung bezeichnet wird, welches gegebenenfalls durch Trocknen, insbesondere Sprühtrocknen oder Eindampfen, vollständig oder teilweise von Wasser und gegebenenfalls vorhandenen Lösemitteln befreit wurde.

Ein weiterer Gegenstand der Erfindung ist somit das Reaktionsprodukt aus dem beschriebenen Verfahren enthaltend das wasserlösliche modifizierte Lignin **ML,** wie vorgängig beschrieben.
Das Reaktionsprodukt kann neben dem wasserlöslichen modifizierten Lignin **ML** weitere Bestandteile enthalten, wie insbesondere nicht umgesetzte Edukte, die eingesetzten Enzyme oder Mediatoren, sowie weiterhin Salze oder Nebenprodukte.
Das Reaktionsprodukt kann ohne weitere Aufarbeitung als Lösung oder in teilweise oder vollständig getrockneter Form verwendet werden, auf die gleiche Weise, wie das wasserlösliche modifizierte Lignin **ML**, wie im Folgenden beschrieben.

Das Reaktionsprodukt ist in getrockneter Form wasserlöslich, das heisst, es löst sich zu mindestens 95 Gewichts-% beim Auflösen von 50 mg in 5 ml deionisiertem Wasser bei 20°C nach 30 Minuten unter Rühren. Bevorzugt löst es sich auf die gleiche Weise auch in einer Pufferlösung bei pH 7 oder tiefer, bevorzugt bei pH 6 oder tiefer, mehr bevorzugt bei pH 5 oder tiefer, am meisten bevorzugt bei pH 4 oder tiefer.

Ein weiterer Gegenstand der Erfindung ist die Verwendung des wasserlöslichen modifizierten Lignins **ML** oder des Reaktionsprodukts enthaltend dieses als Dispergiermittel für Zusammensetzungen enthaltend mindestens ein mineralisches Bindemittel.

Bei der Verwendung als Dispergiermittel wird insbesondere die Verarbeitbarkeit der Zusammensetzung enthaltend mineralische Bindemittel verbessert. Dabei wird die Vermischung der mineralischen Bindemittel mit Wasser so verbessert, dass diese gleichmässiger und einfacher erfolgt. Weiterhin wirkt das wasserlösliche modifizierte Lignin **ML** bzw. das Reaktionsprodukt enthaltend dieses dabei als Verflüssiger in der Zusammensetzung, was einen besonderen Vorteil darstellt. Durch die Wirkung als Verflüssiger wird die Fliessfähigkeit und Verarbeitbarkeit der mineralischen Zusammensetzung erhöht, ohne den Wasseranteil erhöhen zu müssen, was ein Vorteil ist. Oder es kann der Wasseranteil in der Zusammensetzung bei der Verarbeitung reduziert werden, ohne dass die Verarbeitbarkeit darunter leidet, was sehr erwünscht ist. Überraschenderweise verzögert das wasserlösliche modifizierte Lignin **ML** bzw. das Reaktionsprodukt enthaltend dieses das Abbinden der Zusammensetzung deutlich weniger als wenn als Dispergiermittel das wasserunlösliche Lignin **L,** welches für die Herstellung verwendet wurde, eingesetzt wird.

Ein geeignetes mineralisches Bindemittel ist insbesondere ein mineralisches Bindemittel, welches in Anwesenheit von Wasser in einer Hydratationsreaktion zu festen Hydraten oder Hydratphasen reagiert. Dies ist insbesondere ein hydraulisches Bindemittel, welches mit Wasser auch unter Wasser erhärtbar ist, wie insbesondere Zement oder hydraulischer Kalk, oder ein latent hydraulisches Bindemittel, welches unter dem Einwirken von Zusätzen mit Wasser abbindet, wie insbesondere Schlacke, oder puzzolanische Bindemittel wie insbesondere Flugasche, oder ein nicht hydraulisches Bindemittel wie insbesondere Gips in Form von Anhydrit oder Halbhydrat-Gips.
Bevorzugt ist das mineralische Bindemittel ausgewählt aus der Gruppe bestehend aus Zement, Gips, gebrannter Kalk und Flugasche.

Besonders bevorzugt als mineralisches Bindemittel ist Zement, insbesondere ein Zement gemäss Euronorm EN 197-1 oder ein Sulfoaluminatzement oder ein Tonerdeschmelzzement.
Am meisten bevorzugt ist ein Zement enthaltend Portlandzement gemäss EN 197-1.
Ein weiterer Gegenstand der Erfindung ist eine Zusammensetzung, enthaltend
- mindestens ein mineralisches Bindemittel,
- mindestens ein wasserlösliches modifiziertes Lignin **ML,** wie vorgängig beschrieben, und,
- gegebenenfalls Wasser.

Eine solche Zusammensetzung weist bei einem verhältnismässig geringen Wassergehalt eine sehr gute Verarbeitbarkeit auf, insbesondere eine gute Fliessfähigkeit und eine schnelle Abbindezeit.

Das mineralische Bindemittel ist insbesondere ein mineralisches Bindemittel, welches in Anwesenheit von Wasser in einer Hydratationsreaktion zu festen Hydraten oder Hydratphasen reagiert. Dies ist insbesondere ein hydraulisches Bindemittel, welches mit Wasser auch unter Wasser erhärtbar ist, wie insbesondere Zement oder hydraulischer Kalk, oder ein latent hydraulisches Bindemittel, welches unter dem Einwirken von Zusätzen mit Wasser abbindet, wie insbesondere Schlacke, oder puzzolanische Bindemittel wie insbesondere Flugasche, oder ein nicht hydraulisches Bindemittel wie insbesondere Gips in Form von Anhydrit oder Halbhydrat-Gips.
Bevorzugt ist das mineralische Bindemittel ausgewählt aus der Gruppe bestehend aus Zement, Gips, gebrannter Kalk und Flugasche.
Besonders bevorzugt als mineralisches Bindemittel ist Zement, insbesondere ein Zement gemäss Euronorm EN 197-1 oder ein Sulfoaluminatzement oder ein Tonerdeschmelzzement.
Am meisten bevorzugt ist ein Zement enthaltend Portlandzement gemäss EN 197-1.

Das wasserlösliche modifizierte Lignin **ML** wird bevorzugt in einer Menge von 0.05 bis 5 Gewichts-%, besonders bevorzugt 0.1 bis 2 Gewichts-%, insbesondere 0.15 bis 2 Gewichts-%, bezogen auf 100 Gewichts-% minderalisches Bindemittel, eingesetzt.

Das wasserlösliche modifizierte Lignin **ML** ist insbesondere in Form eines Reaktionsprodukts, wie vorgängig beschrieben, vorhanden.

Die Zusammensetzung kann zusätzlich weitere Substanzen enthalten, wie insbesondere die Folgenden:
- Zusatzstoffe, wie sie in mineralischen Bindemittel-Zusammensetzungen üblicherweise verwendet werden, wie insbesondere Silica fume, Hüttensande, Flugasche oder Kalksteinfüller;
- Zuschlagstoffe, wie insbesondere Sand, Kies, Steine, Quarzmehl oder Kreiden;
- Betonverflüssiger, wie insbesondere Ligninsulfonate, sulfonierte Naphthalin-Formaldehyd Kondensate, sulfonierte Melamin-Formaldehyd-Kondensate, Polysaccharide, oder Derivate davon; oder Polycarboxylatether;
- oder weitere Hilfsstoffe, wie insbesondere Beschleuniger, Korrosionsinhibitoren, Verzögerer, Schwindreduzierer, Entschäumer oder Porenbildner.

Die Zusammensetzung wird bevorzugt hergestellt, indem mindestens ein mineralisches Bindemittel und mindestens ein wasserlösliches modifiziertes Lignin **ML** bereitgestellt und anschliessend miteinander vermischt werden.
Weitere Substanzen der Zusammensetzung können dabei vorgängig mit dem mineralischen Bindemittel vermischt und gegebenenfalls gelagert worden sein, oder sie können vorgängig mit dem wasserlöslichen modifizierten Lignin **ML** vermischt und gelagert worden sein, oder sie können kurz vor, beim oder kurz nach dem Vermischen des mineralischen Bindemittels und des wasserlöslichen modifizierten Lignins ML zugegeben und in die Zusammensetzung eingemischt werden.

Durch die Zugabe von Wasser beginnt das Abbinden der Zusammensetzung. Dabei bildet das mineralische Bindemittel eine feste Struktur aus.
Die Menge Wasser wird bevorzugt so gewählt, dass die Zusammensetzung eine für die Verarbeitung gewünschte Konsistenz aufweist. Insbesondere weist die Zusammensetzung eine gute Fliessfähigkeit auf, sodass sie gepumpt oder sonstwie gefördert werden kann und beim Verteilen gut verfliesst. Weiterhin bevorzugt wird mit einer möglichst geringen Wassermenge gearbeitet, sodass nach dem Abbinden nicht zu viel überschüssiges Wasser in der ausgehärteten Zusammensetzung verbleibt, weil das die Festigkeiten reduziert. Die geeignete Menge von Wasser hängt stark von der Art und Feinheit des mineralischen Bindemittels, der Art, Menge und Feinheit der Zuschlagstoffe, der Menge des wasserlöslichen modifizierten Lignins **ML** und gegebenenfalls vorhandenen weiteren Substanzen ab.

Die einer solchen Zusammensetzung zugegebene Wassermenge wird auch als Anmachwasser bezeichnet. Sie wird im Fall von Zement als mineralisches Bindemittel mittels W/Z angegeben. Der W/Z gibt die Menge Wasser in Gramm bezogen auf die Menge an Zement in Gramm an.

Ein weiterer Gegenstand der Erfindung ist ein Formkörper, erhalten durch Abbinden und Aushärten der beschriebenen Zusammensetzung nach deren Kontakt mit Wasser.
Dabei weist der Formkörper eine dreidimensionale Form auf.
Bevorzugt stellt der Formkörper ein Bauteil von einem Gebäude dar, wie insbesondere einen Rohbau, eine Wand, einen Boden, eine Beschichtung, einen Estrich oder eine Füllung.

Ein weiterer Gegenstand der Erfindung ist ein Dispergiermittel für mineralische Bindemittel, enthaltend mindestens ein wasserlösliches modifiziertes Lignin **ML,** wie vorgängig beschrieben.
Das Dispergiermittel kann als Feststoff vorliegen, beispielsweise als Pulver oder Granulat.

Bevorzugt enthält das Dispergiermittel Wasser. Es kann insbesondere als Lösung, Suspension oder Paste vorliegen.
Das Dispergiermittel kann weitere übliche Additive enthalten. Diese können insbesondere andere Verflüssiger, beispielsweise andere modifizierte Lignine, Ligninsulfonate, sulfonierte Naphthalin-Formaldehyd-Kondensate, sulfonierte Melamin-Formaldehyd-Kondensate, Polysaccharide oder Derivate davon oder Polycarboxylatether (PCE) sein. Weitere Zusätze können Stabilisatoren, Antioxidantien, Farbstoffe, Beschleuniger, Verzögerer, Schwindreduzierer, Entschäumer, Luftporenbildner oder Schaumbildner sein.
Bevorzugt weist das Dispergiermittel einen Gehalt an wasserlöslichem modifiziertem Lignin **ML** im Bereich von 5 bis 100 Gewichts-%, insbesondere 10 bis 90 Gewichts-% auf.

Das wasserlösliche modifizierte Lignin **ML** und/oder das Dispergiermittel kann in einer Zusammensetzung enthaltend mindestens ein mineralisches Bindemittel insbesondere verwendet werden als Verflüssiger, zur Verbesserung der Verarbeitbarkeit und/oder zur Verbesserung der Fliessfähigkeit, oder als Wasserreduzierer, also zur Reduktion der Wassermenge bei gleichbleibender Verarbeitbarkeit.

Das wasserlösliche modifizierte Lignin **ML** und/oder das Dispergiermittel kann in festem Aggregatszustand eingesetzt werden, beispielsweise als Pulver oder Granulat. Solche festen Zusätze lassen sich gut transportieren und lagern.
Im festen Aggregatszustand kann das wasserlösliche modifizierte Lignin **ML** ein Bestandteil einer so genannten Trockenmischung, beispielsweise einer Zementzusammensetzung, sein. Eine solche Trockenmischung ist typischerweise in Säcke abgepackt oder wird in Silos gelagert. Sie ist auch nach längerer Lagerungszeit einsetzbar und weist eine gute Rieselfähigkeit auf.

Das wasserlösliche modifizierte Lignin **ML** und/oder das Dispergiermittel kann einer Zusammensetzung enthaltend mindestens ein mineralisches Bindemittel kurz vor oder kurz nach oder zusammen mit der Zugabe des Anmachwassers zugefügt werden. In einer bevorzugten Ausführungsform erfolgt die Zugabe in Form einer wässrigen Lösung oder Dispersion, insbesondere als Anmachwasser oder als Teil des Anmachwassers. Die Herstellung der wässrigen Lösung erfolgt insbesondere durch nachträgliches Vermengen mit Wasser.
Das wasserlösliche modifizierte Lignin **ML** kann einer Zusammensetzung enthaltend mindestens ein mineralisches Bindemittel aber auch vor oder während ihrem Mahlvorgang, beispielsweise dem Mahlprozess von Zementklinker zu Zement, zugegeben werden.

Bei der Verwendung des wasserlöslichen modifizierten Lignins **ML** als Dispergiermittel für mineralische Bindemittel wird ein vorteilhafter verflüssigender Effekt erzielt, ohne dass die Abbindezeit in einem unerwünscht hohen Mass zunimmt.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.
"Ref." steht für "Referenz-Beispiel"

### Beschreibung der Messmethoden:

Das mittlere Molekulargewicht der wasserunlöslichen Lignine und der wasserlöslichen modifizierten Lignine wurde mittels SEC Analyse bestimmt. Die SEC Analyse wurde auf einem HPLC System ausgerüstet mit einer Säulenkaskade der Firma Polymer Standards Service GmbH (MCX 10µm 1000Å, MCX 10µm 100'000Å + Vorsäule) und einem UV-Detektor bei 254 nm durchgeführt. Als Laufmittel und Lösemittel für die Proben wurde 0.01 N wässrige Natronlauge verwendet. Die Messung erfolgte bei 40°C und einem Laufmittelfluss von 0.5 ml/min. Die Kalibration erfolgte mittels 5 engen Polymerstandards aus sulfoniertem Polystyrol im Molekulargewichtsbereich M_{w} von 1'800 bis 400'000 g/mol und p-Toluolsulfonsäure.

Die Menge an organischer Verbindung **V** in der Reaktionsmischung wurde mittels HPLC bestimmt. Aus der Differenz zwischen Menge an organischer Verbindung zu Beginn der Reaktion zur Menge an organischer Verbindung nach einer bestimmten Reaktionszeit wurde der "Reaktionsumsatz in %" berechnet. Messbedingungen bei der HPLC-Analyse:
Laufmittel A: 1.5 g Ameisensäure in 1 I deionisiertem Wasser
Laufmittel B: Acetonitril
Laufmittelfluss 1.5 ml/min
Linearer Gradient von 100% A auf 60% A innerhalb von 20 Minuten.
Säule: Inertsil® ODS-3, 150 mm, 5 µm (GL-Sciences, Japan)
Die Auswertung der Chromatogramme erfolgte mittels UV bei den für die jeweiligen Verbindungen charakteristischen Wellenlängen, für Sulfanilsäure bei 248 nm, für 4-Aminobenzoesäure bei 290 nm und für 5-Amino-2-methylbenzolsulfonsäure bei 268 nm.

### Verwendete wasserunlösliche Lignine L:

Das mittlere Molekulargewicht der Lignine wurde mit der in den Beispielen beschriebenen Methode bestimmt.
**Lignin-1:** Kraftlignin aus Nadelholz (Indulin® AT von MeadWestvaco, USA) mit M_{w} von 6'000 und Mₙ von 2'000
**Lignin-2:** Organosolv-Lignin aus Fichte (Nadelholz; Pilotanlage, Deutschland) mit M_{w} von 6'000 und Mₙ von 2'100
**Lignin-3:** Organosolv-Lignin aus Buche (Laubholz; Pilotanlage, Deutschland) mit M_{w} von 15'400 und Mₙ von 2'400
**Lignin-4:** Graslignin (Biosurfact 8000 von ALM, Indien) mit M_{w} von 4'100 und Mₙ von 1'500
**Lignin-5:** Organosolv-Lignin aus Laubholz (Lignol HP-L™ von Lignol, Canada) mit M_{w} von 6'800 und Mₙ von 2'200.

Der Feuchtegehalt der eingesetzten wasserunlöslichen Lignine wurde mit einem Halogentrockner der Firma Mettler Toledo bestimmt und bei der eingesetzten Menge der Lignine berücksichtigt.

Als "Lignin_Na-Salz-Lösung" wird eine wässrige Lösung bezeichnet, welche hergestellt wurde durch Aufschlämmen einer 10 g trockenes Lignin enthaltenden Menge Lignin in ca. 50 ml deionisiertem Wasser, anschliessender Zugabe von so viel 1N Natronlauge, dass 98 bis 100 Gewichts-% des Lignins in Lösung gingen, und Auffüllen auf 100 ml mittels deionisiertem Wasser. Eine so hergestellte Lignin_Na-Salz-Lösung enthält 100 mg Lignin pro Milliliter.
Als "Lignin_Na-Salz" wird der erhaltene Feststoff nach Trocknen der Lignin_Na-Salz-Lösung bei 70°C in einem Ofen bis zur Gewichtskonstanz bezeichnet.

### Verwendete Enzyme:

### Laccase-Lösung:

Aus Laccase (Laccase aus *Trametes versicolor von* Sigma Aldrich, Schweiz) wurde mit deionisiertem Wasser eine Lösung mit einer Aktivität von 12 U/ml hergestellt.

### Tyrosinase-Lösung:

Aus pilzlicher Tyrosinase (von Sigma Aldrich, Schweiz) wurde mit deionisiertem Wasser eine erste Lösung mit 250 U/ml und eine weitere Lösung mit 5'000 U/ml hergestellt.

### Weitere verwendete Substanzen bzw. Abkürzungen:

### ABTS-Lösung:

Wässrige Lösung des Mediators ABTS (2,2'-Azino-bis(3-ethylbenzthiazoline-6-sulphonsäure) in einer Konzentration von 1 mmol pro Liter.

**Pufferlösung pH** 7: Wässrige Pufferlösung enthaltend 17.65 ml 0.1 M Zitronensäure-Monohydrat-Lösung und 82.35 ml 0.2 M Di-Natriumhydrogenphosphat-Lösung in 100 ml.

**Pufferlösung pH 4:** Pufferlösung pH 4 von Metrohm, Schweiz.

### Herstellung von Reaktionsprodukten enthaltend wasserlösliches modifiziertes Lignin ML:

### Beispiel 1:

In einem 100 ml Rundkolben wurden 73 ml deionisiertes Wasser, 5 ml einer wässrigen Lösung von Sulfanilsäure-Na-Salz (100 mM), 10 ml ABTS-Lösung und 2 ml Lignin-1_Na-Salz-Lösung miteinander vermischt und der pH mittels 1N HCl auf ca. 7 eingestellt. Anschliessend wurden 10 ml Laccase-Lösung zugegeben. Dem Rundkolben wurde ein Rückflusskühler aufgesetzt. Die Reaktionsmischung wurde bei 30-35°C mit einem Magnetrührer während 94 Stunden stark gerührt und von Zeit zu Zeit der Reaktionsumsatz bestimmt, wie in Tabelle 1 angegeben. Anschliessend wurde die Reaktionsmischung ohne weitere Reinigung am Rotationsverdampfer eingeengt. Es wurde ein braun-schwarzer Feststoff mit einem mittleren Molekulargewicht M_{w} von 17'900 g/mol und Mₙ von 4'600 g/mol des darin enthaltenen wasserlöslichen modifizierten Lignins **ML** erhalten.

### Beispiel 2:

Das Beispiel 1 wurde wiederholt, wobei aber anstelle der Lösung von Sulfanilsäure-Na-Salz eine wässrige Lösung von 4-Aminobenzoesäure-Na-Salz (100 mM) eingesetzt wurde. Es wurde ein braun-schwarzer Feststoff mit einem mittleren Molekulargewicht M_{w} von 16'600 g/mol und Mₙ von 3'900 g/mol des darin enthaltenen wasserlöslichen modifizierten Lignins **ML** erhalten.

**Tabelle 1: Umsatz von Sulfanilsäure bzw. 4-Aminobenzoesäure nach den angegebenen Reaktionszeiten**

| | **Beispiel 1** | **Beispiel 2** |
|---|---|---|
| Umsatz nach 22 Stunden | 7 % | 5 % |
| Umsatz nach 46 Stunden | 10 % | 15 % |
| Umsatz nach 70 Stunden | 27 % | 41 % |
| Umsatz nach 94 Stunden | 29 % | 43 % |

### Vergleichsreaktionen ohne Lignin:

Beispiel 1 und Beispiel 2 wurden wiederholt, wobei aber anstelle von deionisiertem Wasser die Pufferlösung pH 7 und statt der Lignin-1_Na-Salz-Lösung Wasser verwendet wurde. Nach 70 Stunden Reaktionszeit wurde der Umsatz von Sulfanilsäure-Na-Salz bzw. 4-Aminobenzoesäure-Na-Salz mit 0% bestimmt. Es hatte also keine Reaktion der Sulfanilsäure oder der 4-Aminobenzoesäure mit der Laccase stattgefunden.

### Vergleichsreaktionen ohne Laccase:

Beispiel 1 und Beispiel 2 wurden wiederholt, wobei aber anstelle der Laccase-Lösung deionisiertes Wasser verwendet wurde. Nach 70 Stunden Reaktionszeit wurde der Umsatz von Sulfanilsäure bzw. 4-Aminobenzoesäure mit 0% bestimmt. Es hatte also keine Reaktion der Sulfanilsäure bzw. der 4-Aminobenzoesäure mit dem Lignin stattgefunden.

### Beispiele 3 bis 6:

Die Beispiele 3 bis 6 wurden wie das Beispiel 1 durchgeführt, wobei aber die Reagenzien gemäss Tabelle 2 in den angegebenen Mengen eingesetzt wurden. Die Reaktionsmischung wurde jeweils am Ende der Reaktion gefriergetrocknet. Es wurde jeweils ein wasserlösliches braun-schwarzes Pulver erhalten.

**Tabelle 2: Eingesetzte Reagenzien in den angegebenen Mengen und Umsatz von Sulfanilsäure bzw. 4-Aminobenzoesäure nach 24h**

| | **Beispiel 3** | **Beispiel 4** | **Beispiel 5** | **Beispiel 6** |
|---|---|---|---|---|
| Deionisiertes Wasser | 29.2 ml | 29.2 ml | 27.2 ml | 29.2 ml |
| ABTS-Lösung (1mM) | 4.0 ml | 4.0ml | 4.0 ml | 4.0 ml |
| Sulfanilsäure-Na-Salz-Lösung (100 mM) | 2.0 ml | - | 2.0 ml | 1.0 ml |
| 4-Aminobenzoesäure-Na-Salz-Lösung (100 mM) | - | 2.0 ml | 2.0 ml | 1.0 ml |
| Lignin-1_Na-Salz-Lösung (100 mg/ml) | 0.8 ml | 0.8 ml | 0.8 ml | 0.8 ml |
| HCl 1N | 0.13 ml | 0.13 ml | 0.13 ml | 0.13 ml |
| Laccase-Lösung (12 U/ml) | 4.0 ml | 4.0 ml | 4.0 ml | 4.0 ml |
| pH der Reaktionsmischung | 5.9 | 6.0 | 6.0 | 6.0 |
| Umsatz Sulfanilsäure / 4-Aminobenzoesäure nach 24 Stunden | 30% | 43% | 18% / 40% | 14% / 44% |

### Beispiel 7:

In einem 250 ml Rundkolben wurden 66.2 ml deionisiertes Wasser, 4.0 ml einer wässrigen Lösung von Sulfanilsäure-Na-Salz (100 mM) und 1.6 ml Lignin-1_Na-Salz-Lösung miteinander vermischt und der pH mittels 1N HCl auf ca. 6 eingestellt. Anschliessend wurden 8.0 ml Laccase-Lösung zugegeben. Der Reaktionskolben wurde mit einem offenen Schliffhahn, versehen mit einem mit Pressluft gefüllten Ballon, verschlossen. Die Reaktionsmischung wurde bei 30-35°C mit einem Magnetrührer 4 Tage stark gerührt. In dieser Zeit wurde der Pressluftballon mehrmals frisch gefüllt. Der Umsatz an Sulfanilsäure nach 4 Tagen betrug 20%. Die Reaktionsmischung wurde ohne weitere Aufarbeitung im Ofen bei 70°C getrocknet. Es wurde ein braun-schwarzer Feststoff mit einem mittleren Molekulargewicht M_{w} von 20'000 g/mol und Mₙ von 8'000 g/mol des darin enthaltenen wasserlöslichen modifizierten Lignins **ML** erhalten. 50 mg davon lösten sich vollständig in 5 ml Pufferlösung pH 4.

Als Vergleich wurde das Beispiel 7 wiederholt, wobei aber anstelle der Sulfanilsäure-Na-Salz-Lösung deionisiertes Wasser zugegeben wurde 50 mg des dabei erhaltenen braun-schwarzen Feststoffs waren nicht löslich in 5 ml Pufferlösung pH 4.

Als weiterer Vergleich wurde die Lignin-1_Na-Salz-Lösung im Ofen bei 70°C getrocknet. 50 mg des dabei erhaltenen braun-schwarzen Feststoffes waren nicht löslich in 5 ml Pufferlösung pH 4.

### Beispiel 8:

In einem 250 ml Rundkolben wurden 30 ml Lignin-1_Na-Salz-Lösung, 4 ml ABTS-Lösung und 30.0 ml deionisiertes Wasser vermischt. Unter Rühren wurden 0.70 g Sulfanilsäure zugegeben. Der pH wurde mittels 1N HCl auf ca. 6 eingestellt. Anschliessend wurden 6.0 ml Laccase-Lösung und 8.0 ml Wasser zugegeben. Der Reaktionskolben wurde mit einem offenen Schliffhahn, versehen mit einem mit Pressluft gefüllten Ballon, verschlossen. Die Reaktionsmischung wurde bei 30-35°C während 72 Stunden mit einem Magnetrührer stark gerührt. Der Pressluftballon wurde während der Reaktion mehrmals frisch gefüllt. Die Reaktionsmischung wurde für ca. 10 min. auf 95°C erhitzt und ohne Reinigung bei 70°C im Ofen getrocknet. Es wurde ein braun-schwarzer Feststoff mit einem mittleren Molekulargewicht M_{w} von 14'600 g/mol und Mₙ von 3'000 g/mol des darin enthaltenen wasserlöslichen modifizierten Lignins **ML** erhalten. 50 mg des erhaltenen Feststoffes waren in 5 ml Pufferlösung pH 4 vollständig löslich.

### Beispiele 9 bis 13:

Für jedes Beispiel wurden 20 ml einer wässrigen Sulfanilsäure-Na-Salz-Lösung (200 mM), 4 ml ABTS-Lösung und 30 ml Lignin_Na-Salz-Lösung der in der Tabelle 3 angegebenen Lignine (Ausgangslignin) in einem Becherglas gemischt. Der pH wurde mittels 0.5 N HCl auf 5.8 eingestellt. Anschliessend wurden 6 ml Laccase-Lösung eingemischt und mit deionisiertem Wasser auf 80 ml aufgefüllt. Die Reaktionsmischung wurde dann in einen 250 ml Rundkolben transferiert und mit einem offenen Schliffhahn, versehen mit einem mit Pressluft gefüllten Ballon, verschlossen. Die Reaktionsmischung wurde bei 30-35°C mit einem Magnetrührer während 72 Stunden stark gerührt. Der Pressluftballon wurde während der Reaktionen mehrmals frisch gefüllt. Die Reaktionsmischungen, welche am Ende der Reaktionszeit nicht klar waren, wurden in 50 ml Zentrifugenröhrchen bei 8'000 rpm zentrifugiert. Die überstehende Lösung und der unlösliche Anteil wurden getrennt und separat bei 70°C im Ofen getrocknet. Der lösliche Anteil des Reaktionsprodukts, der Reaktionsumsatz und die Molekulargewichte des erhaltenen wasserlöslichen modifizierten Lignins **ML** sind in Tabelle 3 angegeben.

**Tabelle 3:**

| | Ausgangslignin | löslicher Anteil des Reaktionsprodukts | Reaktionsumsatz | erhaltenes modifiziertes Lignin ML | |
|---|---|---|---|---|---|
| | | | | M_{w} (g/mol) | Mₙ (g/mol) |
| **Beispiel 9** | Lignin-1 | 100% | 30 % | 28'300 | 4'500 |
| **Beispiel 10** | Lignin-2 | 100% | 36 % | 7'200 | 2'500 |
| **Beispiel 11** | Lignin-3 | 54 % | 56 % | nicht bestimmt | nicht bestimmt |
| **Beispiel 12** | Lignin-4 | 80 % | 47 % | 10'500 | 2'200 |
| **Beispiel 13** | Lignin-5 | 100% | 43 % | 4'600 | 1'900 |

### Beispiel 14:

In einem Becherglas wurden 40 ml Lignin-1_Na-Salz-Lösung, 50 ml einer wässrigen Lösung von Sulfanilsäure-Na-Salz (200 mM) und 20 ml ABTS-Lösung miteinander vermischt und der pH mittels 0.5 N HCl auf 5.9 eingestellt. Anschliessend wurden 30 ml Laccase-Lösung zugegeben und mit deionisiertem Wasser auf 160 ml aufgefüllt. Die Reaktionsmischung wurde in einen 500 ml Einhalskolben transferiert und dieser mit einem offenen Schliffhahn, versehen mit einem mit Pressluft gefüllten Ballon, verschlossen. Die Reaktionsmischung wurde bei 30-35°C mit einem Magnetrührer 70 Stunden stark gerührt und der Pressluftballon während der Reaktion mehrmals frisch gefüllt. Nach 70 Stunden waren 50% der Sulfanilsäure umgesetzt. Die erhaltene klare, dunkelbraune Lösung wurde bei 70°C im Trockenschrank getrocknet. Es wurde ein braun-schwarzer Feststoff mit einem mittleren Molekulargewicht M_{w} von 37'000 g/mol und Mₙ von 4'600 g/mol des darin enthaltenen wasserlöslichen modifizierten Lignins **ML** erhalten. 50 mg des erhaltenen Feststoffes waren in 5 ml Pufferlösung pH 4 vollständig löslich.

### Beispiel 15:

Beispiel 14 wurde wiederholt, wobei aber anstelle der Sulfanilsäure-Na-Salz-Lösung eine 4-Aminobenzoesäure-Na-Salz-Lösung (200 mM) eingesetzt wurde. Nach 70 Stunden waren 45% der 4-Aminobenzoesäure umgesetzt. Die erhaltene klare, dunkelbraune Lösung wurde bei 70°C im Trockenschrank getrocknet. Es wurde ein braun-schwarzer Feststoff mit einem mittleren Molekulargewicht M_{w} von 32'000 g/mol und Mₙ von 4'200 g/mol des darin enthaltenen wasserlöslichen modifizierten Lignins **ML** erhalten. 50 mg des erhaltenen Feststoffs waren in 5 ml Pufferlösung pH 4 teilweise löslich und in Wasser bei pH 6 vollständig löslich.

### Beispiel 16:

Beispiel 14 wurde wiederholt, wobei aber anstelle von 20 ml ABTS-Lösung 20 ml deionisiertes Wasser und anstelle von 30 ml Laccase-Lösung 70 mg Laccase (12 U/mg) als Pulver und 30 ml deionisiertes Wasser verwendet wurden. Nach 24 Stunden waren 25% und nach 48 Stunden 39% der Sulfanilsäure umgesetzt. Die erhaltene klare, dunkelbraune Lösung wurde bei 70°C im Trockenschrank getrocknet. Es wurde ein braun-schwarzer Feststoff mit einem mittleren Molekulargewicht M_{w} von 114'000 g/mol und Mₙ von 7'900 g/mol des darin enthaltenen wasserlöslichen modifizierten Lignins **ML** erhalten. 50 mg des erhaltenen Feststoffs waren in 5 ml Pufferlösung pH 4 vollständig löslich.

### Beispiele 17 bis 20:

Für jedes Beispiel wurden in einem Becherglas 2 ml einer wässrigen Lösung von Sulfanilsäure-Na-Salz (100 mM), 30 ml deionisiertes Wasser und 0.8 ml der jeweiligen Lignin-1_Lösung (100 mg/ml) oder 80 mg Lignin-1-Pulver gemäss Tabelle 4 vermischt. Der pH wurde mittels 1N HCl oder 0.1 N NaOH auf 6.0 eingestellt. Anschliessend wurden 4 ml Laccase-Lösung zugegeben und mit deionisiertem Wasser auf 40 ml aufgefüllt. Die Reaktionsmischung wurde in einen 100 ml Rundkolben transferiert und dieser mit einem offenen Schliffhahn, versehen mit einem mit Pressluft gefüllten Ballon, verschlossen. Die Reaktionsmischung wurde bei 30-35°C mit einem Magnetrührer während mehreren Stunden, wie in Tabelle 4 angegeben, stark gerührt. Der Pressluftballon wurde während der Reaktion mehrmals frisch gefüllt und von Zeit zu Zeit wurde der Reaktionsumsatz bestimmt, wie in Tabelle 4 angegeben.

**Tabelle 4: Art der Zugabe von Lignin-1 und Reaktionsumsatz nach bestimmten Reaktionszeiten**

| | **Beispiel 17** | **Beispiel 18** | **Beispiel 19** | **Beispiel 20** |
|---|---|---|---|---|
| Lignin-1 gelöst in | NaOH^{*} | Aceton/H₂O^{**} | Dioxan/H₂O^{**} | Als Pulver |
| Umsatz nach 21 Stunden | 19 % | 16 % | 14 % | 7 % |
| Umsatz nach 45 Stunden | 23 % | 20 % | 19 % | 12 % |
| Umsatz nach 70 Stunden | 28 % | 21 % | 21 % | 16 % |

| | | | | |
|---|---|---|---|---|
| *) entspricht der Lignin-1_Na-Salz-Lösung **) Volumen-Verhältnis von Lösungsmittel zu Wasser von 4 zu 1 | | | | |

### Beispiel 21:

In einem Becherglas wurden 8.0 ml Lignin-1_Na-Salz-Lösung, 24 ml deionisiertes Wasser und 0.25 g Taurin vermischt. Der pH wurde mittels 1N NaOH auf 9.0 eingestellt. Anschliessend wurden 1.0 ml Tyrosinase-Lösung (5'000 U/ml) zugegeben und mit deionisiertem Wasser auf 40 ml aufgefüllt. Die Reaktionsmischung wurde in einen 250 ml Rundkolben transferiert und dieser mit einem offenen Schliffhahn, versehen mit einem mit Pressluft gefüllten Ballon, verschlossen. Die Reaktionsmischung wurde bei 20-25°C mit einem Magnetrührer während 47 Stunden stark gerührt. Der Pressluftballon wurde während der Reaktion mehrmals frisch gefüllt. Die Reaktionsmischung wurde bei 70°C im Trockenschrank getrocknet. Es wurde ein braun-schwarzer Feststoff mit einem mittleren Molekulargewicht M_{w} von 11'000 g/mol und Mₙ von 3'100 g/mol des darin enthaltenen wasserlöslichen modifizierten Lignins **ML** erhalten. 50 mg des erhaltenen Feststoffes waren in 5 ml Pufferlösung pH 4 teilweise und in 5 ml Pufferlösung pH 7 vollständig löslich.

### Beispiel 22:

In einem konischen Zentrifugenröhrchen mit 40 ml Fassungsvolumen wurden 0.8 ml Lignin-1_Na-Salz-Lösung und 5 ml einer wässrigen Lösung von Histidin (20 mM) vermischt und der pH mittels 1N HCl auf 9 eingestellt. Anschliessend wurden 2 ml Tyrosinase-Lösung (250 U/ml) zugegeben und mit deionisiertem Wasser auf 20 ml aufgefüllt. Das verschlossene Zentrifugenröhrchen wurde bei 25°C und 250 rpm 48 Stunden geschüttelt. Die erhaltene dunkelbraune Reaktionsmischung wurde gefriergetrocknet, wobei ein braun-schwarzer Feststoff erhalten wurde. 50 mg davon waren in Pufferlösung pH 7 vollständig löslich.

### Beispiel 23:

In einem 1-Liter Schikanekolben wurden 20 ml einer wässrigen Lösung von 5-Amino-2-methylbenzolsulfonsäure-Na-Salz (200 mM), 4 ml ABTS-Lösung und 30 ml Lignin-1_Na-Salz-Lösung vermischt und der pH mittels 1N HCl auf 6 eingestellt. Anschliessend wurden 6 ml Laccase-Lösung zugegeben und mit deionisiertem Wasser auf 100 ml aufgefüllt. Die Reaktionsmischung wurde 6 Tage bei 30°C und 200 rpm geschüttelt und von Zeit zu Zeit der Reaktionsumsatz bestimmt, wie in Tabelle 5 angegeben. Die Reaktionsmischung wurde ohne weitere Reinigung gefriergetrocknet, wobei ein schwarz-brauner Feststoff erhalten wurde. 50 mg davon waren in 5 ml Pufferlösung pH 4 vollständig löslich.

**Tabelle 5: Umsatz von 5-Amino-2-methylbenzolsulfonsäure nach gewissen Reaktionszeiten**

| | **Beispiel 23** |
|---|---|
| Umsatz nach 24 Stunden | 59% |
| Umsatz nach 48 Stunden | 81% |
| Umsatz nach 72 Stunden | 86% |
| Umsatz nach 144 Stunden | 95% |

### Verwendung des wasserlöslichen modifizierten Lignins ML als Dispergiermittel:

### Bestimmung der Fliessgrenze:

In einem Kunststoffbecher wurden 25 g Zement mit 9.5 g Wasser für einen W/Z von 0.38 beziehungsweise mit 9.0 g Wasser für einen W/Z von 0.36 von Hand mit einer Spatel 20 Sekunden gut gemischt und so eine Zementpaste erhalten. Für den Fall, dass die Zementpaste ein Dispergiermittel enthalten sollte, war dieses vorgängig in einer Menge von 0.3 Gewichts-% auf 100 % Zement in den 9.5 g bzw. 9.0 g Wasser gelöst worden. Die Zementpaste wurde dann in einen Metallzylinder von 22 mm Durchmesser und 50 mm Höhe gefüllt und der Metallzylinder in der Halterung des Rheometes (Physica MCR 301, Anton Paar, Österreich; Software Rheoplus) befestigt. Eine Metallhelix mit einer Höhe von 32 mm und einem Durchmesser von 20 mm wurde in die Zementpaste versenkt. Die Zementpaste wurde nach einem vorgegeben Scherprofil, wie in Tabelle 6 beschrieben, durch Rotation der Helix geschert und die Scherspannung in Abhängigkeit von der Scherrate aufgezeichnet. Aus der Kurve der abnehmenden Scherrate wurde jeweils, nach ca. 5 Minuten und nach ca. 30 Minuten, bei Scherrate Null die zugehörige Scherspannung in Pascal (Pa) abgelesen, welche die Fliessgrenze der Zementpaste darstellt. Je tiefer die Fliessgrenze umso besser fliesst die Zementpaste.

**Tabelle 6: Scherprofil für die Bestimmung der Fliessgrenze**

| Phase | Anzahl Messpunkte | Dauer pro Messpunkt (s) | Scherrate (s⁻¹) | |
|---|---|---|---|---|
| 1 | 30 | 1 | 100 | Mischen |
| 2 | 90 | 1 | 500 | Vorscheren |
| 3 | 30 | 10......1 log | 0.1-500 log | Zunehmende Scherrate |
| 4 | 30 | 1 .......10 log | 500-0.1 log | Abnehmende Scherrate zur Bestimmung der Fliessgrenze nach ca. 5 Minuten |
| 5 | 100 | 1 | 0.1 | |
| 6 | 20 | 60 | 1 | Mischen langsam |
| 7 | 10 | 1 | 500 | Aufmischen schnell |
| 8 | 30 | 10......1 log | 0.1-500 log | Zunehmende Scherrate |
| 9 | 30 | 1.......10 log | 500-0.1 log | Abnehmende Scherrate zur Bestimmung der Fliessgrenze nach ca. 30 Minuten |
| 10 | 10 | 1 | 500 | Aufmischen schnell |

### Bestimmung der Abbindezeit:

Zur Bestimmung der Abbindezeit wurden nach der Bestimmung der Fliessgrenze ca. 10 g der Zementpaste aus dem Metallzylinder des Rheometers in ein Glasgefäss gefüllt, dieses verschlossen und die zeitliche Entwicklung der Hydratationswärme der Zementpaste mittels isothermer Mikrokalorimetrie (TAM AIR, TA Instruments, USA) verfolgt. Die Abbindezeit stellt dabei die Zeitspanne zwischen dem Vermischen des Zements mit Wasser bis zum Erreichen der maximalen Hydratationstemperatur (nach der Induktionsphase bzw. Ruhephase) dar.

### Beispiel 24 bis 29:

Bestimmung der Fliessgrenze und der Abbindezeit mit Zement CEM I 42.5, Normo 4 (von Holcim, Schweiz) bei einem W/Z von 0.38 mit den Dispergiermitteln gemäss Tabelle 7.

**Tabelle 7: Fliessrenze und Abbindezeit von Zementpasten**

| | verwendetes Dispergiermittel | Fliessgrenze nach ca. 5 Minuten (Pa) | Fliessgrenze nach ca. 30 Minuten (Pa) | Abbindezeit (Stunden) |
|---|---|---|---|---|
| **Ref. 1** | ohne | 126 | 158 | 8.3 |
| **Beispiel 24** | Feststoff aus Beispiel 1 | 29 | 42 | 11.0 |
| **Beispiel 25** | Feststoff aus Beispiel 2 | 29 | 35 | 12.6 |
| **Ref. 2** | Lignin-1_Na-Salz | 29 | 31 | 17.8 |
| **Ref. 3** | ohne | 196 | 242 | nicht bestimmt |
| **Beispiel 26** | Feststoff aus Beispiel 3 | 54 | 81 | nicht bestimmt |
| **Beispiel 27** | Feststoff aus Beispiel 4 | 95 | 124 | nicht bestimmt |
| **Beispiel 28** | Feststoff aus Beispiel 5 | 97 | 56 | nicht bestimmt |
| **Beispiel 29** | Feststoff aus Beispiel 6 | 55 | 92 | nicht bestimmt |

Die erfindungsgemässen wasserlöslichen modifizierten Lignine zeigen bei der Verwendung als Dispergiermittel eine starke Reduktion der Fliessgrenze und damit eine gute Verflüssigungswirkung bei deutlich geringerer Abbindezeit als das für deren Herstellung eingesetzte wasserunlösliche Lignin-1.

Es ist bei der Beurteilung der Resultate in Tabelle 7 wichtig zu berücksichtigen, dass derselbe Zement jeweils nur für eine Messserie (Ref.1 und Beispiel 24 und 25 und Ref. 2; und Ref. 3 und Beispiele 26 bis 29) verwendet wurde, so dass beim Vergleich von Resultaten aus verschiedenen Messserien leichte, durch den Zement aus unterschiedlichen Chargen verursachte Schwankungen in Betracht gezogen werden müssen. Dies erklärt die Abweichungen der Werte von Ref. 1 gegenüber Ref. 3.

### Beispiel 30 und 31:

Bestimmung der Fliessgrenze von Zementpasten hergestellt mit Zement CEM I 42.5 (aus drei Schweizer Zementwerken 1:1:1 in Gewichtsteilen) bei einem W/Z von 0.36 mit den Dispergiermitteln gemäss Tabelle 8. Zum Vergleich wurde ein Na-Ligninsulfonat (Avebene N9 von Avebene, Frankreich) als Dispergiermittel eingesetzt.

**Tabelle 8: Fliessgrenze von Zementpasten**

| | verwendetes Dispergiermittel | Fliessgrenze nach ca. 5 Minuten (Pa) | Fliessgrenze nach ca. 30 Minuten (Pa) |
|---|---|---|---|
| **Ref. 4** | ohne | 147 | 169 |
| **Beispiel 30** | Feststoff aus Beispiel 16 | 84 | 82 |
| **Beispiel 31** | Feststoff aus Beispiel 21 | 41 | 54 |
| **Ref. 5** | Na-Ligninsulfonat | 59 | 64 |

### Herstellung von Zusammensetzungen (Mörtelmischungen):

### Beispiele 32 bis 39:

In einem Zwangsmischer der Firma Hobart wurden 750 g Zement CEM I 42.5 (aus drei Schweizer Zementwerken 1:1:1 in Gewichtsteilen), 141 g Kalksteinfüller (Nekafill 15 der Kalkfabrik Netstal, Schweiz), 738 g Sand 0-1 mm, 1107 g Sand 1-4 mm und 1154 g Sand 4-8 mm 1 Minute trocken gemischt. Innerhalb von 30 Sekunden wurden eine Mischung aus 352.5 g Wasser für W/Z 0.50 (Beispiele 38 und 39) bzw. 382.5 g Wasser für W/Z 0.54 (Beispiele 32 bis 37) und 22.5 g einer mit einem handelsüblichen Entschäumer entschäumten, 10 Gewichts%-igen wässrigen Lösung des Feststoffs aus dem jeweiligen Beispiel, wie in Tabelle 9 und 10 angegeben, zugegeben und noch weitere 2.5 Minuten gemischt. Die Gesamtmischzeit nass dauerte jeweils 3 Minuten. In gleicher Weise wurden Referenz-Mörtel hergestellt, wobei aber 22.5 g einer ebenfalls entschäumten, 10 Gewichts%-igen Lösung des Lignin-1_Na-Salzes oder des Na-Ligninsulfonates (Avebene N9 von Avebene, Frankreich) verwendet wurde. In gleicher Weise wurden auch Mörtelmischungen ohne Dispergiermittel mit 375 g Wasser für W/Z 0.50 bzw. mit 405 g Wasser für W/Z 0.54 als Vergleich hergestellt.

Die Zusammensetzungen wurden folgendermassen geprüft:
Das **Ausbreitmass** wurde gemäss EN 1015-3 bestimmt.
Der **Luftgehalt** wurde gemäss EN 1015-7 gemessen.
Das **Hydratationsverhalten** wurde durch Messen der Temperatur im Verlauf der Zeit nach dem Anmachen mit Wasser ermittelt. Die Temperaturmessung erfolgte unter adiabatischen Bedingungen mit einem Thermoelement als Temperatursensor.
Als **Abbindezeit** wird für diese Beispiele die Zeitspanne zwischen dem Vermischen mit Wasser bis zum Erreichen des nach der Induktionsphase bzw. Ruhephase auftretenden Temperaturmaximums verstrichenen Zeit bezeichnet. Für die Bestimmung der **Druckfestigkeit** der ausgehärteten Mörtel wurden Mörtelprismen von 4x4x16 cm hergestellt und nach dem Ausschalen bei 20°C und 95% relativer Luftfeuchte gelagert. Die Druckfestigkeit nach 24 Stunden und 7 Tagen wurde gemäss EN 196-1 bestimmt.

**Tabelle 9: Eigenschaften von Mörtelmischungen**

| | **Dispergiermittel** | **W/Z** | **Luft-gehalt** | **Ausbreitmass (in mm)** | | |
|---|---|---|---|---|---|---|
| | | | **(%)** | **0 min** | **30 min** | **60 min** |
| **Ref. 6** | ohne | 0.54 | 2.5 | 162 | 152 | 149 |
| **Beispiel 32** | Feststoff aus Beispiel 9 | 0.54 | 2.3 | 176 | 142 | 144 |
| **Beispiel 33** | Feststoff aus Beispiel 10 | 0.54 | 2.4 | 190 | 162 | 148 |
| **Beispiel 34** | wasserlöslicher Teil des Feststoff aus Beispiel 11 | 0.54 | 2.3 | 182 | 152 | 144 |
| **Beispiel 35** | wasserlöslicher Teil des Feststoff aus Beispiel 12 | 0.54 | 2.3 | 162 | 150 | 142 |
| **Beispiel 36** | Feststoff aus Beispiel 13 | 0.54 | 2.4 | 182 | 148 | 138 |
| **Beispiel 37** | Feststoff aus Beispiel 23 | 0.54 | 2.0 | 196 | 175 | 170 |
| **Ref. 7** | Na-Ligninsulfonat | 0.54 | 2.4 | 176 | 146 | 138 |
| **Ref. 8** | Lignin-1_Na-Salz | 0.54 | 2.3 | 174 | 160 | 152 |

**Tabelle 10: Eigenschaften von Mörtelmischungen und Formkörpern**

| | **Dispergiermittel** | **W/Z** | **Luftgehalt (%)** | **Ausbreitmass (in mm)** | | | **Abbindezeit (h)** | **Druckfestigkeit (Pa)** | |
|---|---|---|---|---|---|---|---|---|---|
| | | | | **0 min** | **30 min** | **60 min** | | **1 Tag** | **7 Tage** |
| **Ref. 9** | ohne | 0.50 | 1.4 | 148 | 142 | 140 | 12.8 | 20.7 | 47.2 |
| **Beispiel 38** | Feststoff aus Beispiel 14 | 0.50 | 2.0 | 178 | 156 | 151 | 14.0 | 18.6 | 50.1 |
| **Beispiel 39** | Feststoff aus Beispiel 15 | 0.50 | 2.1 | 166 | 160 | 147 | 14.2 | 18.6 | 50.2 |
| **Ref. 10** | Na-Ligninsulfonat | 0.50 | 2.3 | 175 | 166 | 148 | 14.7 | 17.8 | 46.9 |
| **Ref. 11** | Lignin-1_Na-Salz | 0.50 | 2.4 | 176 | 162 | 156 | 15.7 | 17.5 | 47.6 |

## Patentansprüche

1. Wasserlösliches modifiziertes Lignin **ML,** herstellbar durch enzymatische Umsetzung von mindestens einem wasserunlöslichen Lignin **L** mit mindestens einer organischen Verbindung **V,** welche mindestens eine Gruppe ausgewählt aus primärer oder sekundärer Aminogruppe, Hydroxylgruppe und Phenylgruppe besitzt und ein mittleres Molekulargewicht Mₙ im Bereich von 75 bis 2'500 g/mol aufweist, wobei bei der enzymatischen Umsetzung mindestens ein Enzym, ausgewählt aus Peroxidasen oder Phenoloxidasen. insbesondere Laccasen oder Tyrosinasen, vorhanden ist.

2. Wasserlösliches modifiziertes Lignin **ML** gemäss Anspruch 1, **dadurch gekennzeichnet, dass** die organische Verbindung **V** ausgewählt ist aus der Gruppe bestehend aus
A) Verbindungen der Formel (I),
Zₓ-R-G-R-W_{y} (I)
wobei
Z unabhängig voneinander für -OH, -NH₂, O-R¹ oder R¹ steht,
G für einen Phenylen- oder Naphthylenrest steht,
R unabhängig voneinander für eine kovalente Bindung oder einen linearen oder verzweigten, gegebenenfalls ungesättigte Anteile enthaltenden aliphatischen Kohlenwasserstoffrest mit 1 bis 8 C-Atomen, insbesondere für eine kovalente Bindung, steht,
R¹ unabhängig voneinander für eine Alkylgruppe mit 1 bis 4 C-Atomen steht,
x für 1 oder 2 oder 3 steht,
y für 1 oder 2 oder 3 steht, und
W für -COOM, -SO₃M, -OSO₃M, -PO₃M₂ oder -OPO₃M₂ steht, wobei
M für H, ein Alkalimetall, ein Erdalkalimetall, ein zwei- oder dreiwertiges Metall oder ein organisches oder anorganisches Ammonium steht;
B) Verbindungen der Formel (II) 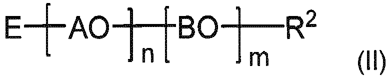 wobei
E für eine Phenoxy- oder eine primäre Aminogruppe steht,
A und B unabhängig voneinander für einen Alkylenrest mit 2 bis 4 C-Atomen stehen,
(n+m) für 1 bis 50 steht, und
R² für H, eine Alkylgruppe mit 1 bis 8 C-Atomen, -COOM , -SO₃M, -OSO₃M, -PO₃M₂ oder -OPO₃M₂ steht;
C) Aminosäuren, insbesondere eine der 21 natürlichen alpha-Aminocarbonsäuren;
und
D) aliphatischen, linearen oder verzweigten Aminosulfonsäuren mit 2 bis 10 C-Atomen.

3. Wasserlösliches modifiziertes Lignin **ML** gemäss Anspruch 2, **dadurch gekennzeichnet, dass** die Verbindung der Formel (I) eine aromatische Verbindung mit mindestens einer Sulfonsäuregruppe oder Carbonsäuregruppe und mindestens einer an den Phenylen- oder Naphthylen-Rest gebundenen Aminogruppe, insbesondere Sulfanilsäure oder 4-Aminobenzoesäure, ist.

4. Wasserlösliches modifiziertes Lignin **ML** gemäss einem der Ansprüche 1 oder 2, **dadurch gekennzeichnet, dass** die organische Verbindung **V** ausgewählt ist aus der Gruppe bestehend aus Glycin, Alanin, Glutaminsäure, Asparaginsäure, Tyrosin, Serin, Histidin und Arginin.

5. Wasserlösliches modifiziertes Lignin **ML** gemäss einem der Ansprüche 1 oder 2, **dadurch gekennzeichnet, dass** die organische Verbindung **V** Taurin ist.

6. Wasserlösliches modifiziertes Lignin **ML** gemäss einem der Ansprüche 1 bis 5, **dadurch gekennzeichnet, dass** bei der enzymatischen Umsetzung pro Gramm wasserunlösliches Lignin **L** 0.4 bis 15 mmol, bevorzugt 0.8 bis 10 mmol, besonders bevorzugt 1 bis 5 mmol, insbesondere 1 bis 3 mmol, organische Verbindung **V** eingesetzt werden.

7. Wasserlösliches modifiziertes Lignin **ML** gemäss einem der Ansprüche 1 bis 6, **dadurch gekennzeichnet, dass** bei der enzymatischen Umsetzung mindestens ein Enzym ausgewählt aus Laccasen oder Tyrosinasen vorhanden ist.

8. Wasserlösliches modifiziertes Lignin **ML** gemäss einem der Ansprüche 1 bis 7, **dadurch gekennzeichnet, dass** die enzymatische Umsetzung in einem Reaktionsmedium enthaltend Wasser und gegebenenfalls mindestens ein organisches Lösemittel durchgeführt wird, wobei der Lösemittelanteil bevorzugt weniger als 10 Gewichts-%, insbesondere weniger als 5 Gewichts-%, bezogen auf die gesamte Reaktionsmischung beträgt und die Umsetzung am meisten bevorzugt ganz ohne organische Lösemittel erfolgt.

9. Wasserlösliches modifiziertes Lignin **ML** gemäss einem der Ansprüche 1 bis 8, **dadurch gekennzeichnet, dass** die enzymatische Umsetzung in Gegenwart eines Mediators, bevorzugt ein Tee- oder Holzextrakt, durchgeführt wird.

10. Verfahren zur Herstellung eines wasserlöslichen modifizierten Lignins **ML** gemäss einem der Ansprüche 1 bis 9, indem ein wasserunlösliches Lignin **L** mit mindestens einer organischen Verbindung **V** enzymatisch umgesetzt wird, wobei die organische Verbindung **V** mindestens eine Gruppe ausgewählt aus primärer oder sekundärer Aminogruppe, Hydroxylgruppe und Phenylgruppe besitzt und ein mittleres Molekulargewicht Mₙ im Bereich von 75 bis 2'500 g/mol aufweist, wobei bei der enzymatischen Umsetzung mindestens ein Enzym, ausgewählt aus Peroxidasen oder Phenoloxidasen, insbesondere Laccasen oder Tyrosinasen, vorhanden ist.

11. Reaktionsprodukt aus dem Verfahren gemäss Anspruch 10 enthaltend das wasserlösliche modifizierte Lignin **ML** gemäss einem der Ansprüche 1 bis 9.

12. Verwendung des wasserlöslichen modifizierten Lignins **ML** gemäss einem der Ansprüche 1 bis 9 oder des Reaktionsprodukts gemäss Anspruch 11 als Dispergiermittel für Zusammensetzungen enthaltend mineralische Bindemittel.

13. Zusammensetzung enthaltend
- mindestens ein mineralisches Bindemittel,
- mindestens ein wasserlösliches modifiziertes Lignin **ML** gemäss einem der Ansprüche 1 bis 9, und
- gegebenenfalls Wasser.

14. Formkörper erhalten durch Abbinden und Aushärten der Zusammensetzung gemäss Anspruch 13 nach deren Kontakt mit Wasser.

15. Dispergiermittel für mineralische Bindemittel, enthaltend mindestens ein wasserlösliches modifiziertes Lignin **ML** gemäss einem der Ansprüche 1 bis 9.

## Claims

1. Water-soluble modified lignin **ML,** preparable by enzymatic reaction of at least one water-insoluble lignin **L** with at least one organic compound **V** which possesses at least one group selected from primary or secondary amino group, hydroxyl group and phenyl group and has an average molecular weight Mₙ in the range from 75 to 2500 g/mol, wherein in the enzymatic reaction there is at least one enzyme selected from peroxidases or phenol oxidases, especially laccases or tyrosinases.

2. Water-soluble modified lignin **ML** according to Claim 1, **characterized in that** the organic compound **V** is selected from the group consisting of
A) compounds of the formula (I),
Zₓ-R-G-R-W_{y} (I)
where
Z independently at each occurrence is -OH, -NH₂, O-R¹ or R¹,
G is a phenylene or naphthylene radical,
R independently at each occurrence is a covalent bond or a linear or branched, aliphatic hydrocarbon radical having 1 to 8 C atoms and optionally comprising unsaturated fractions, and more particularly is a covalent bond,
R¹ independently at each occurrence is an alkyl group having 1 to 4 C atoms,
x is 1 or 2 or 3,
y is 1 or 2 or 3, and
W is -COOM, -SO₃M, -OSO₃M, -PO₃M₂ or -OPO₃M₂, where
M is H, an alkali metal, an alkaline earth metal, a di- or trivalent metal or an organic or inorganic ammonium;
B) compounds of the formula (II) 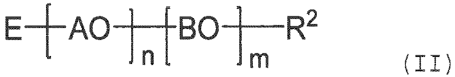 where
E is a phenoxy group or a primary amino group,
A and B independently of one another are an alkylene radical having 2 to 4 C atoms,
(n+m) is 1 to 50, and
R² is H, an alkyl group having 1 to 8 C atoms, -COOM, -SO₃M, -OSO₃M, -PO₃M₂ or -OPO₃M₂;
C) amino acids, more particularly one of the 21 natural alpha-aminocarboxylic acids;
and
D) aliphatic, linear or branched aminosulfonic acids having 2 to 10 C atoms.

3. Water-soluble modified lignin **ML** according to Claim 2, **characterized in that** the compound of the formula (I) is an aromatic compound having at least one sulfonic acid group or carboxylic acid group and at least one amino group bonded to the phenylene or naphthylene radical, more particularly sulfanilic acid or 4-aminobenzoic acid.

4. Water-soluble modified lignin **ML** according to either of Claims 1 and 2, **characterized in that** the organic compound **V** is selected from the group consisting of glycine, alanine, glutamic acid, aspartic acid, tyrosine, serine, histidine and arginine.

5. Water-soluble modified lignin **ML** according to any of Claims 1 or 2, **characterized in that** the organic compound **V** is taurine.

6. Water-soluble modified lignin **ML** according to any of Claims 1 to 5, **characterized in that** the enzymatic reaction is carried out using 0.4 to 15 mmol, preferably 0.8 to 10 mmol, more preferably 1 to 5 mmol, more particularly 1 to 3 mmol, of organic compound **V** per gram of water-insoluble lignin **L.**

7. Water-soluble modified lignin **ML** according to any of Claims 1 to 6, **characterized in that** in the enzymatic reaction there is at least one enzyme selected from laccases or tyrosinases.

8. Water-soluble modified lignin **ML** according to any of Claims 1 to 7, **characterized in that** the enzymatic reaction is carried out in a reaction medium comprising water and optionally at least one organic solvent, the solvent fraction being preferably less than 10 weight%, more particularly less than 5 weight%, based on the total reaction mixture, and the reaction takes place most preferably entirely without organic solvents.

9. Water-soluble modified lignin **ML** according to any of Claims 1 to 8, **characterized in that** the enzymatic reaction is carried out in the presence of a mediator, preferably a tea extract or wood extract.

10. Process for preparing a water-soluble modified lignin **ML** according to any of Claims 1 to 9, by enzymatically reacting a water-insoluble lignin **L** with at least one organic compound **V,** the organic compound **V** possessing at least one group selected from primary or secondary amino group, hydroxyl group and phenyl group and having an average molecular weight Mₙ in the range from 75 to 2500 g/mol, wherein in the enzymatic reaction there is at least one enzyme selected from peroxidases or phenol oxidases, especially laccases or tyrosinases.

11. Reaction product from the process according to Claim 10, comprising the water-soluble modified lignin **ML** according to any of Claims 1 to 9.

12. Use of the water-soluble modified lignin **ML** according to any of Claims 1 to 9 or of the reaction product according to Claim 11 as dispersant for compositions comprising mineral binders.

13. Composition comprising
- at least one mineral binder,
- at least one water-soluble modified lignin **ML** according to any of Claims 1 to 9, and
- optionally water.

14. Shaped body obtained by setting and curing the composition according to Claim 13 after contact thereof with water.

15. Dispersant for mineral binders, comprising at least one water-soluble modified lignin **ML** according to any of Claims 1 to 9.

## Revendications

1. Lignine modifiée **ML** hydrosoluble, pouvant être préparée par réaction enzymatique d'au moins une lignine **L** insoluble dans l'eau avec au moins un composé organique **V** qui comporte au moins un groupe choisi parmi un groupe amino primaire ou secondaire, un groupe hydroxy et un groupe phényle et présente une masse moléculaire moyenne Mₙ dans la plage de 75 à 2 500 g/mole, dans la réaction enzymatique étant présente au moins une enzyme, choisie parmi les peroxydases ou les phénol oxydases, en particulier les laccases ou les tyrosinases.

2. Lignine modifiée **ML** hydrosoluble selon la revendication 1, **caractérisée en ce que** le composé organique **V** est choisi dans le groupe constitué par
A) des composés de formule (I)
Zₓ-R-G-R-W_{y} (I),
dans laquelle
Z représente chaque fois indépendamment un groupe -OH, -NH₂, O-R¹ ou R¹
G représente un radical phénylène ou naphtylène,
R représente chaque fois indépendamment une liaison covalente ou un radical hydrocarboné aliphatique linéaire ou ramifié, contenant éventuellement des fragments insaturés, ayant de 1 à 8 atomes de carbone, en particulier une liaison covalente,
R¹ représente chaque fois indépendamment un groupe alkyle ayant de 1 à 4 atomes de carbone,
x représente 1 ou 2 ou 3,
y représente 1 ou 2 ou 3, et
W représente un groupe -COOM, -SO₃M, -OSO₃M, -PO₃M₂ ou -OPO₃M₂, M représentant H, un métal alcalin, un métal alcalino-terreux, un métal divalent ou trivalent ou un ammonium organique ou inorganique ;
B) des composés de formule (II) 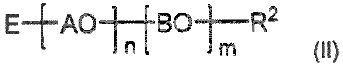 dans laquelle
E représente un groupe phénoxy ou un groupe amino primaire,
A et B représentent indépendamment l'un de l'autre un radical alkylène ayant de 2 à 4 atomes de carbone,
(n+m) représente 1 à 50, et
R² représente H, un groupe alkyle ayant de 1 à 8 atomes de carbone, -COOM, -SO₃M, -OSO₃M, -PO₃M₂ ou -OPO₃M₂ ;
C) des acides aminés, en particulier l'un des 21 acides alpha-aminocarboxyliques naturels ;
et
D) des acides aminosulfoniques aliphatiques, linéaires ou ramifiés, ayant de 2 à 10 atomes de carbone.

3. Lignine modifiée **ML** hydrosoluble selon la revendication 2, **caractérisée en ce que** le composé de formule (I) est un composé aromatique comportant au moins un groupe sulfo ou un groupe carboxy et au moins un groupe amino lié au radical phénylène ou naphtylène, en particulier l'acide sulfanilique ou l'acide 4-amino-benzoïque.

4. Lignine modifiée **ML** hydrosoluble selon l'une quelconque des revendications 1 et 2, **caractérisée en ce que** le composé organique **V** est choisi dans le groupe constitué par la glycine, l'alanine, l'acide glutamique, l'acide aspartique, la tyrosine, la sérine, l'histidine et l'arginine.

5. Lignine modifiée **ML** hydrosoluble selon l'une quelconque des revendications 1 et 2, **caractérisée en ce que** le composé organique **V** est la taurine.

6. Lignine modifiée **ML** hydrosoluble selon l'une quelconque des revendications 1 à 5, **caractérisée en ce que** dans la réaction enzymatique on utilise par gramme de lignine **L** insoluble dans l'eau 0,4 à 15 mmoles, de préférence 0,8 à 10 mmoles, de façon particulièrement préférée 1 à 5 mmoles ; en particulier 1 à 3 mmoles, de composé organique **V.**

7. Lignine modifiée **ML** hydrosoluble selon l'une quelconque des revendications 1 à 6, **caractérisée en ce que** dans la réaction enzymatique est présente au moins une enzyme choisie parmi les laccases ou les tyrosinases.

8. Lignine modifiée **ML** hydrosoluble selon l'une quelconque des revendications 1 à 7, **caractérisée en ce que** la réaction enzymatique est effectuée dans un milieu réactionnel contenant de l'eau et éventuellement au moins un solvant organique, la proportion de solvant étant de préférence inférieure à 10 % en poids, en particulier inférieure à 5 % en poids, par rapport au mélange réactionnel total, et la réaction s'effectuant de façon tout particulièrement préférée sans solvant organique.

9. Lignine modifiée **ML** hydrosoluble selon l'une quelconque des revendications 1 à 8, **caractérisée en ce que** la réaction enzymatique s'effectue en présence d'un médiateur, de préférence un extrait de thé ou de bois.

10. Procédé pour la préparation d'une lignine modifiée **ML** hydrosoluble selon l'une quelconque des revendications 1 à 9, par mise en réaction par voie enzymatique d'une lignine **L** insoluble dans l'eau avec au moins un composé organique **V,** le composé organique **V** comportant au moins un groupe choisi parmi un groupe amino primaire ou secondaire, un groupe hydroxy et un groupe phényle et présentant une masse moléculaire moyenne Mₙ dans la plage de 75 à 2 500 g/mole, dans lequel lors de la réaction enzymatique est présente au moins une enzyme, choisie parmi les peroxydases ou les phénol oxydases, en particulier les laccases ou les tyrosinases.

11. Produit de réaction du procédé selon la revendication 10, contenant la lignine modifiée **ML** hydrosoluble selon l'une quelconque des revendications 1 à 9.

12. Utilisation de la lignine modifiée **ML** hydrosoluble selon l'une quelconque des revendications 1 à 9 ou du produit de réaction selon la revendication 11 en tant que dispersant pour des compositions contenant des liants minéraux.

13. Composition contenant
- au moins un liant minéral,
- au moins une lignine modifiée **ML** hydrosoluble selon l'une quelconque des revendications 1 à 9, et
- éventuellement de l'eau.

14. Corps moulé obtenu par prise et durcissement de la composition selon la revendication 13 après son contact avec de l'eau.

15. Dispersant pour liants minéraux, contenant au moins une lignine modifiée **ML** hydrosoluble selon l'une quelconque des revendications 1 à 9.
